# EUROPEAN PATENT SPECIFICATION

(11) **EP 2 234 564 B1**
(45) Date of publication and mention of the grant of the patent: **30.05.2012**
(21) Application number: 09702489.7
(22) Date of filing: 14.01.2009
(51) Int. Cl.: A61F 2/44

(54) **INTERVERTEBRAL CAGE AND VERTEBRAL FUSION DEVICE**
ZWISCHENWIRBELKÄFIG UND WIRBELFUSIONSVORRICHTUNG
CAGE INTERVERTEBRALE ET DISPOSITIF DE FUSION VERTEBRALE

(30) Priority: 17.01.2008 FR 0850286
(43) Date of publication of application: 06.10.2010
(73) Proprietor: Warsaw Orthopedic, Inc., Warsaw, IN 46581 (US)
(72) Inventor: ASSAKER, Richard, B-7540 Kain (BE); DUPONT, Phillipe, F-77410 Clay Souilly (FR); D'AMORE, Jean-Francois, F-62780 Stella Plage (FR); LEMAITRE, Philippe, 01170 Crozet (FR); MANGIONE, Paolo Fernando, Dr., 33600 Pessac (FR)
(74) Representative: Viering, Jentschura & Partner
(86) International application number: PCT/US2009/030921
(87) International publication number: WO 2009/091775

(56) References cited:
- WO-A-97/15246
- WO-A-99/63914
- WO-A2-2007/098288
- FR-A- 2 815 846
- US-A- 5 683 394
- US-A1- 2002 169 508
- US-A1- 2006 195 100
- US-A1- 2007 255 416

## Description

The invention relates to an intervertebral cage according to the preamble of claim 1.

Accordingly, the invention generally relates to orthopaedic surgery of the vertebral column. More precisely, it relates to devices referred to as "intervertebral fusion cages."

When an intervertebral disk of a patient is deteriorated as the result of disease or trauma, one of the solutions for remedying the pain and difficulty undergone by the patient consists in removing the damaged disk and in carrying out a fusion of the vertebrae involved by means of a device consisting of a cage containing a bone graft, placed between the vertebrae. By growing and solidifying, the graft rigidly connects the vertebral end-plates facing each other.

This solution is particularly indicated in the cervical region of the spine, where the vertebrae are of small dimensions, but may be used elsewhere in the spine.

Two techniques pertaining to this principle are currently in use. In the first technique, the damaged disk is removed, and then the two vertebral bodies are drilled by the anterior pathway, so as to make cavities in them with roughly circular contours facing each other. Then a cage is inserted between the vertebrae, into these cavities, this cage having a roughly circular shape with a wall with multiple perforations, and which contains a bone graft, the growth and solidification of which make it possible to carry out the vertebral fusion through the perforations of the cage. This technique makes it possible, in principle, to perform a good fusion. But it has the disadvantage of laying the cage on the spongy parts of the vertebrae, on account of the preliminary drilling of the vertebral bodies. The consequence of this is that the dimensional stability of the inter-disk space is not guaranteed during the fusion process, because the spongy parts run the risk of not having the solidity necessary for avoiding a coming-together of the vertebrae.

A second technique consists in removing the damaged disk, and carrying out at first only a mild abrasion of the vertebral endplates so as to preserve the cortical matter on the periphery of their respective outer surfaces. Then insertion is made by the anterior pathway of a cage of generally parallelepiped shape open on its upper and lower surfaces, and containing a bone graft, unless this is inserted into the cage later or through a lateral opening made in the cage. The cage is supported on the cortical parts and can be anchored there, in particular if the edges present teeth. The dimensional stability of the inter-disk space is better than in the preceding technique, since the cage is supported by cortical parts which present greater rigidity than the spongy parts. However, the bone fusion is not as good as in the preceding technique.

US 2006/0 195 100 A1 discloses an intervertebral cage according to the preamble of claim 1.

WO 99/63914 discloses a rectangular cage with an empty space therein, and with an end portion defining flanges.

FR-2 815 846-A1 discloses a cylindrical implant having a collar at an axial end, engaged by ring.

US 2007/02 554 16 A1 discloses an intervertebral implant having upper and lower walls and lateral walls extending therebetween, and having an inner chamber.

WO 2007/098288 A2 discloses an intervertebral implant comprising a spacer configured for insertion between vertebral, and a keel formed on upper and lower surfaces of the spacer.

Document US-A-6 235 059 describes an improvement of this last technique. It presents an intervertebral cage of roughly parallelepiped shape, the lateral walls of which can be modelled so as to fit the shape of the vertebral endplates. At least one surface of the cage presents a groove through which, after placement of the cage, a bone graft can be inserted to fill the interior space of the cage and achieve vertebral fusion. The cage is then rigidly connected to a plate fixed on its anterior surface, and the plate itself is fixed by means of screws to the vertebrae to be fused. In this way better dimensional stability of the intervertebral space is obtained, by a combination of the actions of the plate and the cage. As a variant, the plate may be integrated by construction with the cage.

Document US-A-2004 0034430 describes an intervertebral cage of parallelepiped general shape, open on its upper and lower surfaces, but initially comprising only three lateral walls. The fourth lateral wall, which is the anterior wall of the cage, is fixed to the rest of the cage, by means of screws, only after insertion of the cage in the intervertebral space. After fixation of the anterior wall, the cage is entirely closed. Insertion of the bone graft may be carried out before closure of the cage, or after this closure through grooves made in the lateral walls of the cage.

This last configuration has, however, the disadvantage of not guaranteeing a very precise initial positioning for the cage in the intervertebral space at the time of its emplacement, and also of not guaranteeing the preservation of this positioning during all the rest of the emplacement process and carrying-out of the fusion. In particular, there is a risk that the cage will be too deeply placed in the intervertebral space and will overflow into the medullary canal, with the risk of injuring the spinal cord. Conversely, between its installation and the fusion, the cage also risks migration in the anterior direction of the spine and, therefore, of overflowing outside the intervertebral space and no longer being totally supported by the peripheral cortical bone of the vertebral endplates. This risk is particularly significant in the cervical region, on account of the small dimensions of the vertebrae and intervertebral spaces, requiring a very precise positioning of the device and a guaranteed maintenance of this positioning. In fact, US-A-2004 0034430 principally recommends use of its device in the lumbar and thoracic regions of the spine, and between vertebrae L5 and S1.

An objective of the invention is to propose a new intervertebral cage for bone fusion, useful in particular in the cervical region of the spine, guaranteeing both excellent bone fusion and easy positioning at the time of its installation and remaining permanently satisfactory at the time of subsequent steps in the vertebral fusion process.

To this end, the invention has for subject matter an intervertebral cage according to claim 1. Further embodiments of the invention are described in the dependent claims. It may comprise on at least one of the walls of its base element back stop barbs.

Said plate may be rigidly connected to the vertebrae to be fused by screws.

Said plate may at rest present a curvature in its longitudinal direction and/or in its transverse direction, and in that it presents properties of elasticity making possible a straightening of said curvature at the time of installation of the plate.

The plate may present a curvature in its two longitudinal and transverse directions.

Said anchoring means may be anchoring studs made of one piece with the plate.

The anchoring studs may present back stop ridges or back stop barbs.

The closure element may comprise depressions or orifices into which bone graft may penetrate.

The invention also has for subject matter a vertebral fusion device for the carrying-out of the fusion of more than two adjacent vertebrae, characterised in that it comprises several intervertebral cages of the preceding type, and in that they are all closed by a single closure element made up of a plate extending over all vertebrae to be fused.

As pointed out, the invention rests on the use of an intervertebral cage of parallelepiped general shape, for containing a bone graft, initially delimited by three walls and open on its anterior surface. According to the invention, the anterior ends of the lateral walls of the cage comprising the stops designed to come in contact with the anterior edges of the vertebral end-plates of the vertebrae involved in the fusion, at the time of emplacement of the cage by an anterior approach.

More preferably, the back stop barbs worked into the upper and/or lower edges of the walls prevent the cage from tending to be extracted from the intervertebral space in the posterior-anterior direction.

It is only after this emplacement and in situ introduction of the bone graft that the cage is closed by the emplacement of a fourth wall functioning as closure element for the cage.

It may also be made up of a cervical plate that is fixed by various means (screws, studs, etc.) to the vertebrae involved in the fusion. Advantageously, this plate contributes to the compression of the bone graft and to the maintenance of the cage by tending to bring together the vertebrae to which it is fixed, by its own elasticity.

The invention will be better understood upon reading the following description, given with reference to the following appended figures:
- Figure 1 which shows in perspective view an example of an intervertebral fusion cage according to the invention, lacking its closure element;
- Figure 2 which shows this same example of a cage fitted with a closure element not forming part of the invention made up of a plate clipped to the anterior ends of the lateral parts of the cage;
- Figure 3 which shows this same example of a cage fitted with a closure element made up of a rigid plate designed to be screwed into the vertebrae involved in the fusion;
- Figure 4 which shows this same example of a cage fitted with a closure element made up of an elastic plate curved in its longitudinal and transverse directions, anchored to the vertebrae involved in the fusion by means of studs, to be emplaced in a straightened state and tending then to regain its initial curved state, so as to exert a force for bringing the vertebrae together to compress the bone graft present in the cage.

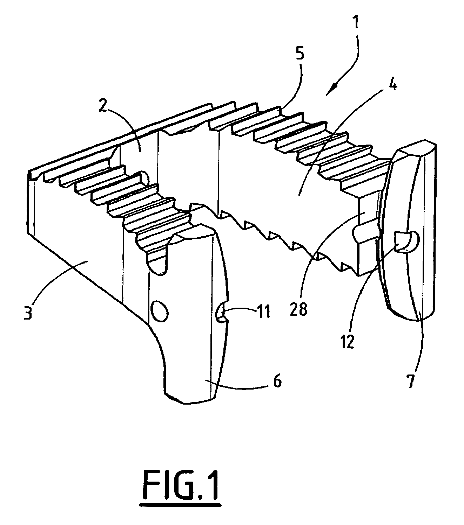

Figure 1 shows an example of base element 1 of the cage according to the invention. It is made up of three walls, each presenting a certain height and a certain thickness, namely:
- a posterior wall 2;
- and two lateral walls 3, 4.

The dimensions of element 1 and its various parts are designed so that after emplacement by the anterior pathway of element 1 following resection of the deteriorated disk, the posterior wall 2 may be in contact with the posterior edges of the endplates of the vertebrae involved and the lateral walls 3, 4 may be in contact with the lateral edges of these same endplates. These edges of the endplates making up areas of relatively high density, the dimensional stability of the intervertebral space after the installation of the cage is ensured to the best.

As shown, the height of walls 2, 3, 4 may not be uniform over its entire length, so as to best restore the natural geometry of the intervertebral space wherein the cage is to be placed.

The upper and lower surfaces of walls 2, 3, 4 are, in the example shown, provided with back stop barbs 5. When the cage is in place, they tend to anchor to the vertebral endplates and their orientation toward the anterior part of the cage opposes the extraction of the latter in the posterior-anterior direction. Optionally, such barbs 5 could be provided for only on some surfaces of some of the walls 2, 3, 4.

According to the invention, the anterior ends of lateral walls 3, 4 are each fitted with a stop 6, 7. These stops have a height appreciably greater than that of walls 3, 4, and therefore greater than that of the intervertebral space in which the cage is to be inserted. In this way, at the time of insertion of element 1 of the cage between the vertebrae involved, the progression of element I is interrupted as soon as stops 6, 7 come in contact with the anterior walls of the vertebrae. If the lengths of lateral walls 3, 4 have been, beforehand, correctly chosen as a function of the morphology of the patient, the surgeon is thus certain that the posterior wall 2 is supported by the posterior edges of the vertebral bodies. Thus it is avoided:
- that element 1 comes out into the medullary cavity at the risk of injuring the spinal cord; this as well at the time of insertion of the element as at the time of the subsequent steps of installation of the cage and at the time of movements later performed by the patient;
- or conversely that insertion of element 1 is not significant enough and that posterior wall 2 is then supported by areas of low density of the vertebral endplates.

On account of the fact that element 1 is open on its anterior surface at this stage, it is not useful to install the bone graft in it prior to its insertion. This facilitates installation of the cage in an overall way. This also presents the advantage that preparation of the vertebral endplates by removal of the cortical bone from the area where they will be in contact with the graft, for the purpose of optimal realization of bone fusion over the entire free surface of the endplates, may be carried out although element 1 is already in place. The tool with which the cortical bone is removed cannot therefore accidentally penetrate into the medullary canal, because the posterior wall 2 of element 1 forms a barrier to the tool. Possible damage of the spinal cord is therefore avoided at this stage. Moreover, it is not necessary to maintain a diversion force between the vertebrae at this stage, management of the intervertebral space being firmly ensured by element 1 alone.

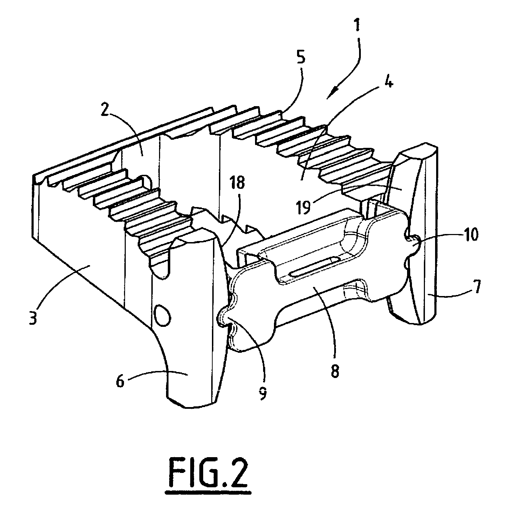

After installation of the bone graft, the cage according to the invention is closed by installation of an anterior wall which may be of any type ensuring maintenance of the graft in the intervertebral space. Figures 2 to 4 show various examples of such closure means.

In the case of Figure 2, closure of the cage is ensured by an anterior wall 8 brought back on element 1, the height of which is comparable to that of the other walls 2, 3, 4 of the cage. To this end, the anterior wall 8 presents lugs 9, 10 which are placed in corresponding housings 11, 12 worked into the anterior ends of lateral walls 3, 4. Maintenance in place of the anterior wall 8 may be ensured, in this example, by a simple elastic deformation of wall 8 which presses lugs 9, 10 into their housings 11, 12. Of course, the positions of lugs 9, 10 and housings 11, 12 could be reversed. Also, in addition to or in place of this elastic deformation of the whole of anterior wall 8, this maintenance in place could be ensured by other means, such as elastic claws placed on the anterior wall 8 or on the lateral walls 3, 4, or by small-size screws.

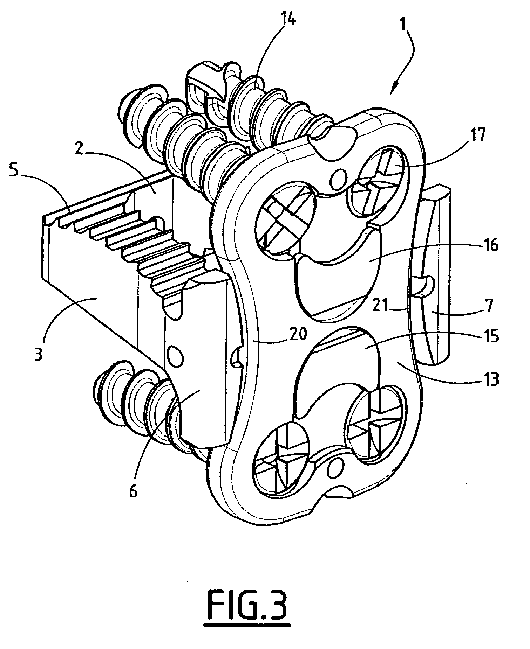

In the case of Figure 3, closure of the cage according to the invention is ensured by a rigid plate 13, of a type known in itself. It is designed to be placed overlapping the two vertebrae to be fused, and is fixed to them by screws 14 penetrating into said vertebrae. Plate 13 may advantageously be equipped, as shown, with devices 15, 16 known in themselves which, after being screwed, can be moved to cover heads 17 of screws 14 and prevent them from tending to be progressively expelled from their housings during the patient's lifetime. Plate 13 makes it possible to keep the vertebrae fixed with respect to each other. It is not necessary that it be rigidly fixed to element 1. In the example shown, the lower edges 18, 19 of stops 6, 7 are of non-rectilinear profile and correspond to the shape of portions 20, 21 of the longitudinal edges of plate 13. In this way, the relative positionings of element 1 and plate 13 are predetermined and maintained over time in an ensured way.

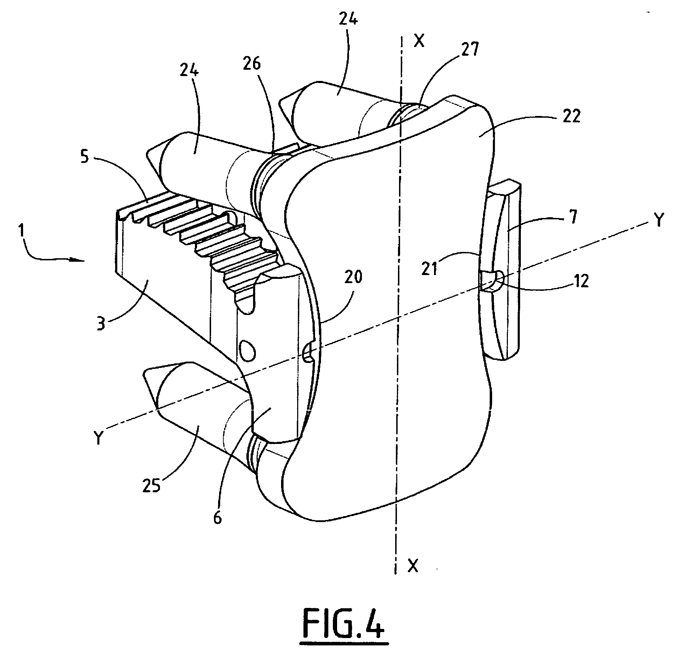

In the case of Figure 4, in order to stopper the cage use is made of a plate 22, as described in detail in the patent application FR 08 50285 under the name of the applicant.

This plate 22 has the distinctive characteristic of presenting at rest a curvature in its longitudinal direction and/or in its transverse direction, and elasticity properties making possible a straightening of said curvature at the time of installation of plate 22. After installation of plate 22, it tends to regain its initial shape.

Plate 22 presents a curvature in its longitudinal direction (XX) which, at rest, gives plate 22 a curved shape in this direction.

Plate 22 may be elastically deformed by means of an appropriate instrument, so as to reduce, indeed eliminate this curvature at the time of installation of the plate. It is after the withdrawal of the instrument that plate 22 tends to regain its initial form, without however totally regaining it, on account of the resistance it ends up encountering on the part of the vertebrae and the cage.

In the example shown, plate 22 also comprises a curvature in its transverse direction (YY) in order to better fit the form of the anterior surfaces of the vertebrae, a curvature which may also be reduced or eliminated at the time of elastic deformation of the plate at the time of its installation.

Plate 22 comprises on its rear surface anchoring studs 23, 24, 25, four in number, in the example shown (three are visible in Figure 4). These studs 23, 24, 25 are more preferably and as shown made of one piece with plate 22: it is therefore not necessary to provide for separate parts such as screws for fixation of plate 22 to the vertebrae, which simplifies installation of plate 22 and management of the stock of parts. Studs 23, 24, 25 have, in the example shown in Figure 4, a roughly elliptical cross-section. It could be circular, or flattened as well to optimise support on the spongy bone. In the example shown, studs 23, 24, 25 each comprise a localised bulge 26 presenting a ridge 27 in proximity to the rear surface of plate 22. This ridge plays the role of an back stop barb and tends to prevent spontaneous extraction of the plate after its emplacement. As a variant, one or more true barbs could be provided on studs 23, 24, 25 to make up this back stop device.

The various dimensions of plate 22 are chosen as a function of the size of the vertebrae involved, the future emplacement of plate 22 and the mechanical properties of the material making it up. It is necessary for plate 22 to present sufficient elasticity so that its curvature in the longitudinal direction (XX) and optionally in its transverse direction (YY) might be greatly reduced, indeed eliminated, prior to installation. In this way, after its installation plate 22 will tend to regain its initial shape without recovering it totally, in such a way that studs 23, 24, 25 will exert an increased force on the walls of their respective orifices with respect to what would occur if plate 22 were not elastically deformable at the time of its installation. Thus the resistance of plate 22 to being ripped out is increased.

Studs 23, 24, 25 are not, more preferably, parallel at rest, but present a convergence in one or two directions of space which makes tearing out plate I after its installation even more difficult.

Moreover, on account of the initial curvature of plate 1 in its longitudinal direction (XX), the tendency of plate 1 to return to its initial shape has the effect of tending to bring together the vertebrae on which plate 1 is installed. Thus, permanent constraint of the bone graft is effectuated, the bone graft having been inserted beforehand into element I of the cage according to the invention. The fusion of the vertebrae is accelerated because of it. In the cervical area, where the invention finds a privileged but not exclusive example of application, studs 23, 24, 25 are provided which are sufficiently long so that a coming together of the vertebrae takes place in their posterior parts. In this way, the natural lordosis of the spine in the area under consideration is restored.

Installation of plate 22 takes place as follows.

The surgeon makes, in the vertebrae under consideration, orifices which will make it possible to initiate insertion of anchoring studs 23, 24, 25. The distances between the axes of the orifices correspond to the distances between studs 23, 24, 25 when plate 1 is in the straightened state.

Then, by means of an appropriate instrument, the surgeon takes hold of plate 1 and exerts a force on it that reduces or eliminates its longitudinal curvature along (XX) (and optionally its curvature along (YY)), so as to give to the ends of studs 23, 24, 25 inter-axis distances corresponding to those of the orifices. In this position, studs 23, 24, 25 are found more preferably with roughly parallel orientations to facilitate their introduction.

Then the surgeon places anchoring points 23, 24, 25 in the orifices of the vertebrae, and impacts plate 22, for example by striking the instrument lightly with a hammer, which straightens it and maintains it, in such a way as to drive the anchoring studs 23, 24, 25 into their orifices, which they themselves enlarge so as to be blocked in them. The rear surface of plate 22 thus comes in contact with the vertebrae.

Then the straightening force on plate 22 is interrupted, for example by disengaging the instrument from plate 22. The latter therefore tends to regain its initial curved shape, which makes the tearing out of plate 22 more difficult for the reasons that have been related. Also, plate 22 permanently exerts a bringing-together force on the vertebrae and compresses the bone graft. Thus the possibilities and the velocity of fusion of the vertebral endplates are increased with respect to what would occur in the absence of compression. The localization and intensity of this compression are controlled by the choice of materials and the dimensions of the plate.

Depressions or orifices in which bone graft can penetrate at the time of its compression can be provided on the rear surface of plate 22. By solidifying, this graft will contribute to maintaining plate 22 in place. These orifices may cross plate 1. Such depressions or orifices could also be present in other means of closure of the cage (wall 8 or plate 13). But the possibility of strongly compressing the graft by means of plate 22 makes this configuration particularly effective from this point of view.

By way of non-restrictive example, it is possible to use a PEEK plate designed to be implanted by the anterior pathway overlapping two cervical vertebrae, of length 22 mm, of maximum width 16 mm and of thickness 1.8 mm. Anchoring studs 23, 24, 25 have a length of 13 mm and a diameter of 3.5 mm, which goes to 4 mm at ridge 27. Their angle of convergence is 6o.

When plate 22 extends overlapping two consecutive vertebrae, the presence of four anchoring studs 23, 24, 25 is generally advised. It is possible to provide for only two of them, in particular if plate 22 is designed to be implanted on the smallest cervical vertebrae. It would also be possible to provide a higher number of studs 23, 24, 25 for plates 22 intended to be implanted in the biggest vertebrae of the spine as a complement to an element 1, to make up a cage according to the invention.

It would also be conceivable to not integrate anchoring elements with plate 22 but to carry out this anchoring by means of, for example, screws crossing orifices worked into plate 22, on the condition of ensuring that the forces exerted by plate 22 to tend to return to its initial curved configuration are properly transmitted to the anchoring elements by means of a sufficient contact surface with the screws, in particular with their threaded rods. The tendency for return of plate 22 in curved configuration makes it possible to increase the friction between the rods of the screws and the walls of their orifices, which makes removal of the screws more difficult and may make it possible to eliminate the anti-withdrawal devices which are ordinarily associated with them.

Also in the case of plate 22, it is possible to give to the lateral edges 20, 21 of plate 22 and to the internal edges of stops 6, 7 rectilinear shapes not requiring a rigorous longitudinal positioning of plate 22 with respect to element 1 at the time of its installation.

Plates 13, 22 of Figures 3 and 4 may also advantageously be stopped against surfaces 28 contrived by withdrawal of stops 6, 7 to the anterior ends of lateral walls 3, 4 of element 1. They contribute in this way to preventing a withdrawal of element I outside the intervertebral space during the patient's lifetime.

A single configuration of element 1 may, as shown, be designed for adaptation to several types of closure elements. This simplifies the manufacturing range and the management of parts by the manufacturer and the user.

The materials that can be used to make up the various parts of the cage according to the invention are those usually used for these applications: stainless steel, titanium and other biocompatible metals, polymers such as PEEK, carbon fibres, etc. In the case in which a biotransparent material is used, non-biotransparent reference points must be arranged in places where they will be useful for verifying the proper positioning of the device at the time of an X-ray.

It is also conceivable to use a plate 13, 22 extending over more than two consecutive vertebrae and which is rigidly connected to them, in such a way that a single such plate 13, 22 works with a plurality of elements 1, each being placed between two of the vertebrae involved.

## Claims

1. Intervertebral cage intended to contain a bone graft for the fusion of two adjacent vertebrae, having a U-shaped base element (1) made of a posterior wall (2) and of two lateral walls (3, 4) connected to it, and a closure element initially separated from the base element (1) and brought on the base element (1), wherein the closure element is a plate (13, 22) to be fixed on the vertebrae to be fused, wherein said base element (1) has, at the anterior ends of its lateral walls (3, 4), stops (6, 7) having a height superior to the heights of said anterior ends, intended to contact the anterior walls of the vertebrae, and wherein the base element includes surfaces (28) configured to align the closure element and joined base element, providing alignment of the closure element laterally between the stops (6, 7) and anteriorly such that the front of the closure element substantially aligns with anterior limits of the stops (6, 7), **characterised in that** the stops (6, 7) have inside edges (18, 19) of non-rectilinear profile in the height direction of the stops (6, 7) and correspond to the shapes of portions (20, 21) of longitudinal edges of the plate (13, 22) whereby the relative positioning of the base element (1) and the plate (13, 22) are predetermined and maintained over time in an ensured way.

2. Intervertebral cage according to claim 1, wherein it has on at least one of the walls (2, 3, 4) of its base element (1) back-stop barbs (5).

3. Intervertebral cage according to claim 1, wherein the plate (13) is fixed on the vertebrae to be fused by screws (14).

4. Intervertebral cage according to claim 1 or 2, wherein the plate (22) has at rest a curvature according to its longitudinal direction (XX), and wherein it has elasticity properties which allow said curvature to be straightened before the plate (22) is set on the vertebrae.

5. Intervertebral cage according to claim 7, wherein the plate (22) has a curvature according both its longitudinal (XX) and transversal (YY) directions.

6. Intervertebral cage according to one of claims 1 to 5, wherein the closure element (8, 13, 22) has depressions or orifices into which bone graft can penetrate.

7. Vertebral fusion device for performing the fusion of more than two adjacent vertebrae, wherein it has several intervertebral cages according to one of claim 1 or 2, and wherein they are all closed by a same closure element made of a plate straddling over all the vertebrae to be fused.

## Patentansprüche

1. Zwischenwirbelkäfig, der vorgesehen ist, ein Knochentransplantat für die Fusion von zwei benachbarten Wirbeln zu enthalten, aufweisend ein U-förmiges Basiselement (1), das aus einer posterioren Wand (2) und aus zwei damit verbundenen lateralen Wänden (3, 4) gebildet ist, und ein Verschlusselement, das anfangs von dem Basiselement (1) getrennt ist und auf das Basiselement (1) aufgebracht wird, wobei das Verschlusselement eine Platte (13, 22) zum Befestigen an den zu fusionierenden Wirbeln ist, wobei das Basiselement (1) an den anterioren Enden seiner lateralen Wände (3, 4) Anschläge (6, 7) aufweist, die eine Höhe haben, die größer als die Höhen der anterioren Enden ist, die vorgesehen sind, um die anterioren Wände der Wirbel zu kontaktieren, und wobei das Basiselement Flächen (28) aufweist, die eingerichtet sind, um das Verschlusselement und das angefügte Basiselement auszurichten, wobei eine Ausrichtung des Verschlusselements lateral zwischen den Anschlägen (6, 7) und anterior bereitgestellt wird, so dass die Vorderseite des Versahlusselements im Wesentlichen zu den anterioren Abgrenzungen der Anschläge (6, 7) ausgerichtet ist, **dadurch gekennzeichnet, dass** die Anschläge (6, 7) Innenränder (18, 19) mit einem nicht geradlinigen Profil in der Höhenrichtung der Anschläge (6, 7) aufweisen und mit den Formen von Abschnitten (20, 21) von Längsrändern der Platte (13, 22) korrespondieren, wodurch die relative Positionierung des Basiselements (1) und der Platte (13, 22) vorbestimmt ist und auf eine gesicherte Weise über die Zeit aufrechterhalten wird.

2. Zwischenwirbelkäfig gemäß Anspruch 1, wobei er an mindestens einer der Wände (2, 3, 4) seines Basiselements (1) Gegenhalter-Widerhaken (5) aufweist.

3. Zwischenwirbelkäfig gemäß Anspruch 1, wobei die Platte (13) an den zu fusionierenden Wirbeln mittels Schrauben (14) befestigt ist.

4. Zwischenwirbelkäfig gemäß Anspruch 1 oder 2, wobei die Platte (22) in Ruhestellung eine Krümmung gemäß ihrer Längsrichtung (XX) aufweist, und wobei sie Elastizitätseigenschaften hat, die zulassen, dass die Krümmung begradigt wird, bevor die Platte (22) auf die Wirbel gesetzt wird.

5. Zwischenwirbelkäfig gemäß Anspruch 7, wobei die Platte (22) eine Krümmung gemäß sowohl ihrer Längsrichtung (XX) als auch ihrer Querrichtung (YY) aufweist.

6. Zwischenwirbelkäfig gemäß einem der Ansprüche 1 bis 5, wobei das Verschlusselement (8, 13, 22) Vertiefungen oder Öffnungen aufweist, in die Knochentransplantat eindringen kann.

7. Wirbelfusionsvorrichtung zum Durchführen der Fusion von mehr als zwei benachbarten Wirbeln, wobei sie mehrere Zwischenwirbelkäfige gemäß einem der Ansprüche 1 oder 2 aufweist, und wobei alle durch dasselbe Verschlusselement verschlossen sind, das aus einer Platte gebildet ist, die sämtliche zu fusionierende Wirbel überspannt.

## Revendications

1. Cage intervertébrale destinée à contenir un greffon osseux pour la fusion de deux vertèbres adjacentes, ayant un élément de base en forme de U (1) fait d'une paroi postérieure (2) et de deux parois latérales (3, 4) reliées à celle-ci, et un élément de fermeture initialement séparé de l'élément de base (1) et placé sur l'élément de base (1), où l'élément de fermeture est une plaque (13, 22) qui doit être fixée sur les vertèbres devant être fusionnées, où ledit élément de base (1) possède, au niveau des extrémités antérieures de ses parois latérales (3,4), des butées (6, 7) ayant une hauteur supérieure aux hauteurs desdites extrémités antérieures, destinées à entrer en contact avec les parois antérieures des vertèbres, et où l'élément de base comprend des surfaces (28) configurées de façon à aligner l'élément de fermeture et l'élément de base raccordé, assurant l'alignement de l'élément de fermeture latéralement entre les butées (6, 7) et antérieurement de sorte que l'avant de l'élément de fermeture soit sensiblement aligné avec les limites antérieures des butées (6, 7), **caractérisée en ce que** les butées (6, 7) possèdent des bords intérieurs (18, 19) de profil non rectiligne dans la direction de la hauteur des butées (6, 7) et correspondent aux formes des parties (20, 21) des bords longitudinaux de la plaque (13, 22), grâce à quoi le positionnement relatif de l'élément de base (1) et de la plaque (13, 22) est prédéterminé et maintenu à long terme d'une manière assurée.

2. Cage intervertébrale selon la revendication 1, laquelle possède sur au moins l'une des parois (2, 3, 4) de son élément de base (1) des ergots anti-recul (5).

3. Cage intervertébrale selon la revendication 1, où la plaque (13) est fixée sur les vertèbres devant être fusionnées par des vis (14).

4. Cage intervertébrale selon la revendication 1 ou 2, où la plaque (22) présente au repos une courbure selon sa direction longitudinale (XX), et laquelle cage possède des propriétés d'élasticité qui permettant de redresser ladite courbure avant que la plaque (22) soit placée sur les vertèbres.

5. Cage intervertébrale selon la revendication 7, où la plaque (22) présente une courbure à la fois selon sa direction longitudinale (XX) et sa direction transversale (YY).

6. Cage intervertébrale selon l'une des revendications 1 à 5, où l'élément de fermeture (8, 13, 22) possède des dépressions ou des orifices dans lesquels le greffon osseux peut pénétrer.

7. Dispositif de fusion vertébrale destiné à réaliser la fusion de plus de deux vertèbres adjacentes, lequel dispositif possède plusieurs cages intervertébrales selon l'une des revendications 1 ou 2, et dans lequel elles sont toutes fermées par un même élément de fermeture fait d'une plaque chevauchant toutes les vertèbres devant être fusionnées.
